(19) Europäisches Patentamt
European Patent Office
Office européen des brevets

(11) Publication number: **0 223 230 B1**

## EUROPEAN PATENT SPECIFICATION

(45) Date of publication of patent specification: 23.10.91

(51) Int. Cl.5: **C12N 15/23**, C12N 15/85

(21) Application number: 86115971.3

(22) Date of filing: 18.11.86

(54) **Process for producing cell having high productivity of protein and process for preparing protein by using the cell.**

(30) Priority: 20.11.85 JP 260450/85

(43) Date of publication of application:
27.05.87 Bulletin 87/22

(45) Publication of the grant of the patent:
23.10.91 Bulletin 91/43

(84) Designated Contracting States:
CH DE FR GB LI

(56) References cited:
EP-A- 0 117 059
EP-A- 0 123 372

JOURNAL OF MOLECULAR AND APPLIED GE-
NETICS, vol. 1, no. 3, 1981, New York, US; G.
RINGOLD et al, "Co-Expression and Amplifi-
cation of Dihydrofolate Reductase cDNA and
the Escherichia coli XGPRT Gene in Chinese
Hamster Ovary Cells"

(73) Proprietor: KANEGAFUCHI KAGAKU KOGYO
KABUSHIKI KAISHA
2-4 Nakanoshima 3-chome
Kita-ku Osaka-shi Osaka-fu(JP)

(72) Inventor: Asahi, Takashi
10-6, Tsutsujigaoka 3-chome§Tarumi-ku
Kobe-shi Hyogo-ken(JP)
Inventor: Nakagawa, Kaku
2-63, Okihama-cho§Takasago-cho
Takasago-shi Hyogo-ken(JP)
Inventor: Niwa, Hideo
2-63, Okihama-cho§Takasago-cho
Takasago-shi Hyogo-ken(JP)
Inventor: Kakutani, Tetsu
Manhaimukakogawa No. 626§835-1, Befu
Befu-cho Kakogawa-shi Hyogo-ken(JP)
Inventor: Kawaharada, Hajime
2183-4, Shinzaike§Hiraoka-cho
Kakogawa-shi Hyogo-ken(JP)
Inventor: Watanabe, Kiyoshi
15-41, Matsugaoka 5-chome
Akashi-shi Hyogo-ken(JP)

PROCEEDINGS OF THE NATIONAL ACADEMY OF SCIENCES OF THE UNITED STATES OF AMERICA, vol. 80, no. 15, August 1983, Baltimore, US; S. J. SCAHILL et all, "Expression and characterization of the product of human immune interferon cDNA gene in Chinese hamster ovary cells, pp. 4654-4658

NUCLEIC ACIDS RESEARCH, vol. 11, no. 3, February 11, 1983, Oxford, GB; J. HAYNES et al, "Constitutive, long-term production of-human interferons by hamster cells containing multiple copies of a cloned interferon gene", pp. 687-706

NATURE, vol. 275, no. 5681, October 19, 1978, New York, London; C. S. Y. CHANG et al, "Phenotypic expression in E. coli of a DNA sequence coding for mouse dihydrofolgate reductase, pp. 617-624

MOLECULAR AND CELLULAR BIOLOGY, vol. 2, no. 11, November 1982, Washington, DC, US; R. J. KAUFMAN et al, "Construction of a Modular Dihydrofoldate Reductase cDNA Gene: Analysis of Signals Utilized for Efficient Expression", pp. 1304-1319

(74) Representative: Türk, Gille, Hrabal
Brucknerstrasse 20
W-4000 Düsseldorf 13(DE)

## Description

The present invention relates to a process for producing cells having a high productivity of a desired protein and a process for preparing the desired protein by employing the cell.

In recent years, the production of desired protein, the protein which is not produced in the host cell, by the gene recombination technique holds great public attention in the field of bioengineering. Among the processes for the production of the desired protein, numerous methods wherein the gene coding for the desired protein is introduced into bacteria such as E. coli and Bacillus subtilis or yeast to express the desired protein have hitherto been developed and the fundamental technique thereof have been almost established.

However, the production of the desired protein by employing higher eucaryotic cells, especially mammalian cells, as a host still has many problems to be solved for the practical application although the method, recently especially, has become to hold public attention due to its many advantages such as that the protein is produced in a natural form as in a living body by glycosylation, various modifications and the like, unlike the protein produced by the method employing bacteria as the host. Examples of such problems are, for instance, that the produced amount of the desired protein is still small as compared with bacteria or yeast, that the host mammalian cells are still limited which can be industrially employed, and the like. Hitherto, various methods employing the mammalian cells as the host have been attempted for producing a large amount of the desired protein in an industrial scale. Among them, the following two methods employing two kinds of host-vector system have been attempted more often.

The first is the method wherein DNA containing bovine papilloma virus gene as a vector and the breast cancer cells of mouse as a host are employed [For example, G. N. Pavlakis et al., Proc. Natl. Acad. Sci. USA, 80, p 397 (1983); T.V. Kamabhadvan et al., Proc. Natl. Acad. Sci. USA, 81, p 6701 (1984); R. Fukunaga et al., Proc. Natl. Acad. Sci. USA, 81, p 5086 (1984)]. Although this method has an advantage that a copy number of the vector DNA is large (from 20 to 100) and thus the desired gene product can be produced in a relatively large amount, it has also disadvantages such as difficulty in maintaining the increase of the produced amount of the product after the transformant is obtained and much restriction for the selection of the host which can be employed.

The second is the method wherein DHFR-deficient Chinese hamster ovary cell line (hereinafter referred to as "CHO(dhfr⁻) cells") [G. Urlab and L. Chasin, Proc. Natl. Acad. Sci. USA, 77, p 4216 (1980)] as a host and a vector containing DHFR cDNA of mouse [A. C. Y. Chang et al., Nature, 275, p 617 (1978); Kaufman et al., Mol. and Cell. Biol., 2, p 1304 (1982)] are employed. According to the method, the transformant can be easily selected by the expression of the introduced DHFR cDNA and the transformant is cultured in a medium containing methotrexate (hereinafter referred to as "MTX") to amplify DHFR cDNA in the cell with a large copy number [G. Ringold el al., J. Mol. Appl. Gen., 1, p 165 (1981)], thereby also amplifying the gene encoding the desired protein adjacent to DHFR cDNA to improve the productivity of the desired protein [For example, J. Haynes and C. Weissman, Nucleic Acids Res., 11, p 687 (1983); S. J. Scahill et al., Proc. Natl. Acad. Sci. USA, 80, p 4654 (1983); Goeddel et al., Japanese Unexamined Patent Publication No. 42321/1984; Y. Chernajovsky et al., DNA, 3, p 297 (1984)]. Although this method has an advantage that a produced amount of the desired protein can be increased by the selection of resistant cells to a high concentration of MTX, it has also a disadvantage that the usable animal cell of the host is limited to only CHO(dhfr⁻) cells at present and CHO-KI cells, which are parental cell line of CHO(dhfr⁻) cells, and other mammalian cells cannot be employed. It is supposed that such disadvantage is due to the following reasons. That is, since wild type cell has a gene coding for its own normal DHFR in the nuclear chromosome and the DHFR protein produced by the gene coding for normal DHFR has less affinity to MTX than DHFR protein of mouse produced by mouse DHFR cDNA, the transformant cannot be selected by MTX in the case of transfection with a vector containing mouse DHFR cDNA. Further, a resistance to a higher concentration of MTX is apt to be attained by the amplification of the chromosomal DHFR gene.

In recent years, for overcoming the above-mentioned disadvantage, various methods have been attempted for preparing DHFR gene encoding DHFR protein with less affinity to MTX than DHFR protein in the host cell. For example, an attempt to obtain mutant DHFR gene from mutant cell line, CHO A 29 cells [M. Wigler et al., Proc. Natl. Acad. Sci. USA, 77, p 3567 (1980); J. K. Christman et al., Proc. Natl. Acad. Sci. USA, 79, p 1815 (1982)] and an attempt to obtain the mutant DHFR gene from mutant fibroblast cell of mouse (A.D. Levinson et al., Japanese Unexamined Patent Publication No. 192089/1984) are known. However, these methods have still difficulty in the practical application since a high concentration of MTX is required for the selection of the transformant and the gene amplification procedure further increases the MTX concentration, which will negatively affect the growth of the host cell.

The present inventors made an earnest study to develop a method to produce higher eucaryotic cells

3

which yield desired protein in high concentration by employing genetic materials which is available to everyone wherein any higher eucaryotic cells can be transformed in so far as the cells show sensitivity to MTX and the desired protein gene can be easily amplified in a large amount by the subsequent gene amplification procedure. As a result, is was found that, if higher eucaryotic cells of the host are transfected with DNA which contains, on the same or different vector, mouse DHFR cDNA and a gene encoding the protein necessary for the selection of the transformant together with the gene encoding the desired protein and the transformant is selected with dominant-acting marker, not with mouse DHFR cDNA, the cell capable of stably producing a large amount of the desired protein can be obtained by culturing the transformant in a medium containing MTX even though the transformant contains normal DHFR gene in chromosomal DNA.

In accordance with the present invention, there are provided a process for producing cells having a high productivity of a desired protein, which comprises (I) transfecting a higher eucaryotic cell which contains a chromosomal DNA coding for dihydrofolate reductase sensitive to methotrexate and functions normally with a gene I coding for a desired protein, a gene II coding for dihydrofolate reductase derived from a wild type cell and a gene III coding for a protein necessary for the selection of a transformant, and (2) culturing the transformant in the presence of a competitive inhibitor to the dihydrofolate reductase activity to amplify the gene I together with the amplification of the gene II, and a process for preparing a desired protein by culturing the thus obtained cells having a high productivity of a specific protein.

Fig. I shows a simple illustration of a cleavage map of plasmid pBR-γ8.6-I which contains the human chromosomal interferon-γ DNA.

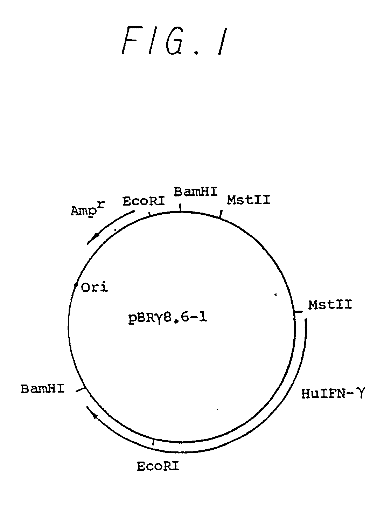

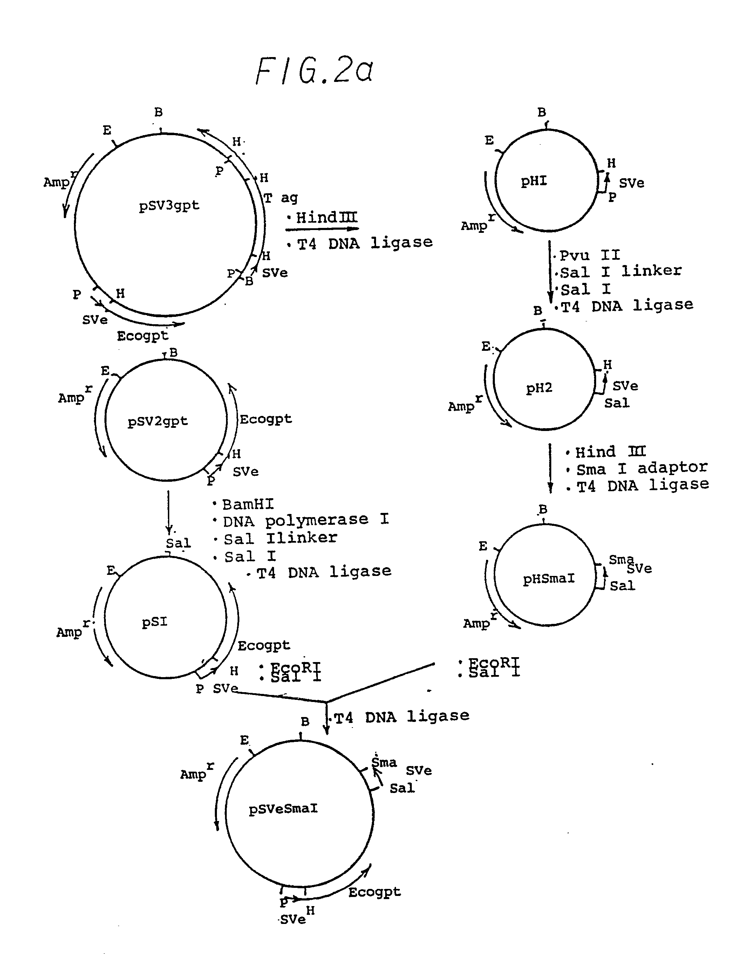

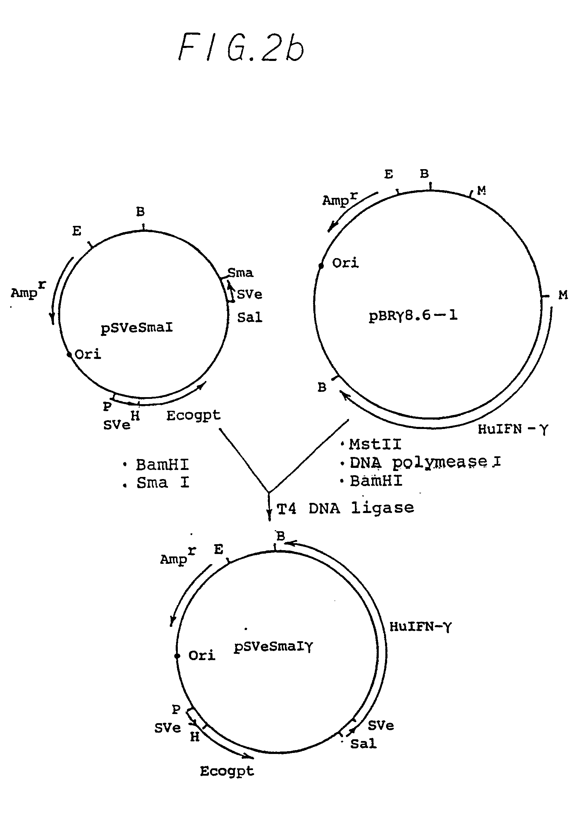

Fig. 2a and Fig. 2b schematically show the construction of the plasmid pSVeSmaI-γ.

In Figs. 2a and 2b, the abbreviations E, B, H, P, Sma, Sal and M show the site of the restriction enzymes EcoRI, BamHI, HindIII, PstI, SmaI, SalI and MstII, respectively.

Abbreviations Amp$^r$, T ag, SVe, Ecogpt and Ori mean ampicillin resistant gene, large T-antigen of SV40, early promoter region of SV40, guanine phosphoribosyl transferase and the replication origin, respectively.

Fig. 3 shows a simple illustration of a cleavage map of plasmid pSVedhfr-γ2 which contains the genes coding for interferon-γ, DHFR and Ecogpt on the same vector, and a construction of pSVedhfr-γ2 from pSVeSmaIγ and pSV2dhfr.

Fig. 4 shows a simple illustration of a cleavage map of plasmid pSVedhfr-γI which contains the genes coding for interferon-γ and DHFR on the same vector, and a construction of pSVedhfr-γI from pSVeSmaIγ and pSV2dhfr.

The mechanism, wherein the gene coding for DHFR and the gene coding for the desired protein adjacent to the gene coding for DHFR are amplified in the host chromosomal DNA by culturing the host cell in a medium containing MTX, is described in some literatures [For example, J. L. Biedler et al., Cancer Res., 32, p I53 (I972); F. W. Alt. et al., J. Biol. Chem., 253, p I357 (I978); J. Christman et al., Proc. Natl. Acad. Sci. USA, 79, p I8I5 (I982); G. Ringold et al., J. Mol. Appl. Gen., I, p I65 (I98I)]. By means of the above citation of the literatures, the description of the mechanism is incorporated into the specification.

As the competitive inhibitor to DHFR, MTX is usually employed. MTX may be usually contained in a medium at a concentration ranging from I0 to 500000 nM. However, in so far as effective for the amplification of DHFR cDNA, the MTX concentration may not be limited to the above upper limit and lower limit and other folic acid derivatives such as homofolic acid or other compound can also be employed. As the higher eucaryotic cell of the host containing normal DHFR gene, CHO-KI cells and MOPC cells are described in the following Examples. However, the present invention is not limited to these cells and many other cell lines such as BHK, VERO, COS, CEM and Namalva can also be employed. Similarly, although interferon-γ is described in the following Examples as the desired protein, other proteins may also be employed such as erythropoietin, tissue plasminogen activator, interleukin 2 and interferon-β. As. the gene encoding the protein necessary for the selection of the transformant, the gene coding for Ecogpt derived from E. coli [R. C. Mulligan and P. Berg, Proc. Natl. Acad. Sci. USA, 78, p 2072 (I98I)] is exemplified in the following Examples. However, any gene can be employed such as Neo$^r$ gene derived from transposon Tn 5 of bacteria [P. J. Southern and P. Berg, J. Mol. Appl. Gen., I, p 327 (I982)] and TK gene derived from herpes virus against thymidine kinase deficient host cells [M. Wigler et al., Cell, II, p 223 (I977)] in so far as the transformant can be selected. Similarly, although DHFR cDNA of mouse is exemplified in the following Examples as the gene coding for DHFR derived from wild type cell, it goes without saying that the DHFR cDNA or chromosomal DNA derived from other animal cells can also be employed.

The present invention is more specifically described and explained by the following Examples. However, it should be understood that the present invention is not limited to such Examples and various changes and modifications can be made without departing from the scope of the present invention.


Example I

4

[Cloning of the human interferon-γ chromosomal DNA]

Human chromosomal DNA obtained from healthy adults' leucocytes was digested with restriction enzyme BamHI, and about 8 to 9 kilobases DNA fragments (hereinafter referred to as "Kb") were prepared with sucrose density-gradient centrifugation. λ -Phage vector charon 28A (available from Bethesda Research Lab. USA) was digested with BamHI, and thereto the BamHI fragments of human chromosomal DNA of 8 to 9 Kb were ligated. The ligated DNA was subjected to the in vitro packaging technique [L. Enquist et al., Methods in enzymology, 28, p 281 (1979)] to form plaques of recombinant phages with the use of Escherichia Coli LE392 as a host. Human interferon-γ gene bearing recombinant phage clones were selected out according to the plaque hybridization technique [W. D. Benthon et al., Science, 196, p 180 (1977)] using the synthesized oligonucleotides CTTGGCTGTTAC, CCTGGCAGTAAC and GCTCTTCGACCTCG as probes.

Four phage clones, S8-II, SI8-6, SI9-5 and S20-I to be hybridized with all of the used three probes, were obtained out of about 2 million recombinant phages. DNA of phage clone S8-II containing human interferon-γ gene was digested with BamHI, was ligated to a BamHI site of plasmid pBR322 (ATCC 31344), and was transformed to E. coli C600 r⁻m⁻. A transformant containing a plasmid wherein the human interferon-γ gene of about 8.6 Kb had been inserted into the BamHI site of pBR322 was selected out, and this plasmid was designated as pBRγ8.6-I (Fig. I).

Example 2
[Preparation of pSVeSmalγ]

Plasmid pSVeSmalγ which has the sequence of human interferon-γ chromosomal DNA ligated to SV40 promoter region was prepared according to the procedures shown in Fig. 2a and Fig. 2b by employing pBRγ8.6-I, pSV2gpt (ATCC 37145) and pSV3gpt (ATCC 37144) [R. C. Mullingen et al., Science, 209, p 1422 (1980)] as starting materials.

pSV3gpt was digested with HindIII and the largest DNA fragment was made cyclic with T4 DNA ligase to form pHI. The PvuII site of pHI was changed to the SalI site with the use of SalI linker to form pH2. Further, the HindIII site of pH2 was changed to the SmaI site with the use of HindIII-SmaI adaptor to form pHSmal.

pSV2gpt was digested with BamHI and the termini were filled to make blunt ends with DNA polymerase I (Klenow). A SalI site was introduced with the use of SalI linker and T4 DNA ligase to form pSI. The pSI was digested with SalI and EcoRI, and an ampicillin-resistant gene bearing DNA fragment was ligated to the SalI-EcoRI fragment of pHSmal having SV40 promoter region to form pSVeSmal [Fig. 2a].

pBRγ8.6-I was digested with MstII and, after the termini were made blunt with DNA polymerase I (Klenow), was digested with BamHI to give the human interferon-γ chromosomal DNA bearing fragment. The obtained DNA fragment was then introduced into pSVeSmal which was digested with SmaI and BamHI, to prepare pSVeSmalγ [Fig. 2b]. The above procedures (Examples I and 2) are precisely described in the specification of European Patent Application No. 0I67852.

Example 3
[Construction of plasmid pSVedhfr-γ2]

For the construction of plasmid pSVedhfr-γ2 which has the genes coding for human chromosomal interferon-γ, DHFR and Ecogpt on the same vector, plasmid pSVeSmalγ prepared in Example 2 and pSV2dhfr (ATCC 37146) were employed as the plasmid vector containing interferon-γ gene and Ecogpt gene and as the plasmid vector containing DHFR cDNA of mouse, respectively.

A solution of I0 μg of pSV2dhfr DNA dissolved in I00 μℓ of a mixture of I0 mM Tris-HCℓ (pH 7.5), 7 mM MgCℓ₂, 60 mM NaCℓ and 7 mM 2-mercaptoethanol was reacted with I0 units of restriction enzyme PvuII at 37°C for 60 minutes. The reaction solution was then heated at 65°C for I0 minutes to inactivate PvuII. After precipitation treatment with 2.5 times amount of ethanol, the resultant was dissolved in a total amount of 25 μℓ of a solution containing 20 mM Tris-HCℓ (pH 7.5), 6 mM MgCℓ₂, 5 mM dithiothreitol and 500 μM ATP. To the solution was added I μg of BamHI linker wherein 5′ end is phosphorylated and thereto I0 unit of T4 DNA ligase was further added to conduct the linkage reaction at 4°C for I7 hours. After completion of the reaction, DNA was collected by the precipitation treatment with ethanol and then dissolved in a total amount of 20 μℓ of a solution containing 20 mM Tris-HCℓ(pH 7.5), I0 mM MgCℓ₂, I0 mM dithiothreitol and 50 mM NaCℓ. To the solution were added 5 units of restriction enzyme BamHI and the digestion reaction was carried out at 37°C for 60 minutes. After the reaction solution was heated at

65°C for 10 minutes to inactivate the enzyme, purification with agarose gel electrophoresis was conducted to give about 1.5 μg of about 1.9 Kb DNA fragment containing DHFR gene.

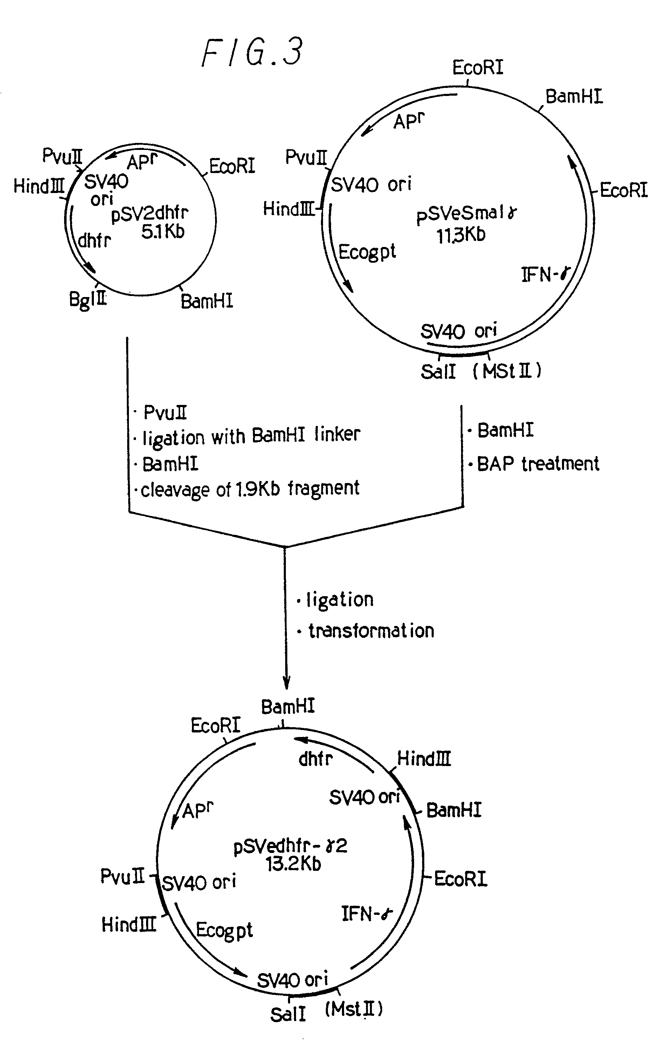

On the other hand, 2 μg of pSVeSmalγ was dissolved in 20 μl of the above BamHI reaction solution and the digestion reaction was conducted. After alkaline phosphatase treatment to remove phosphate residue at the 5' end, the resultant was subjected to the treatment with phenol and the precipitation treatment with ethanol. The obtained DNA was dissolved in T4 DNA ligase reaction solution and thereto a total amount of the above 1.9 Kb DNA fragment was added to dissolve. To the solution were added 5 units of T4 DNA ligase and the linkage reaction was carried out at 4°C for 17 hours. By employing the obtained mixture of recombinant plasmid, E. coli HB 101 (ATCC 33694) was transformed in the usual way to give ampicillin-resistant (Amp$^r$) colony. Plasmid was isolated from the culture solution of the colony to give pSVedhfr-γ2 as shown in Fig. 3.

Example 4
[Construction of plasmid pSVedhfr-γ1]

Plasmid pSVedhfr-γ1 which has the genes coding for interferon-γ and DHFR on the same vector was constructed in the following manner. To a solution of 5 μg of pSV2dhfr DNA dissolved in a total amount of 50 μl of a solution containing 10 mM Tris-HCl (pH 7.5), 7mM MgCl$_2$, 100 mM KCl, 7 mM 2-mercaptoethanol and 0.01 % bovine serum albumin (BSA), each 5 units of the restriction enzymes Pvu I and Hpa I were added and the digestion reaction was conducted at 37°C for 60 minutes. After completion of the reaction, the reaction solution was head at 65°C for 10 minutes to inactivate Pvu I and Hpa I. The reaction solution was then subjected to the purification with agarose gel electrophoresis to give about 3.2 μg about of 3.6 Kb DNA fragment containing DHFR gene.

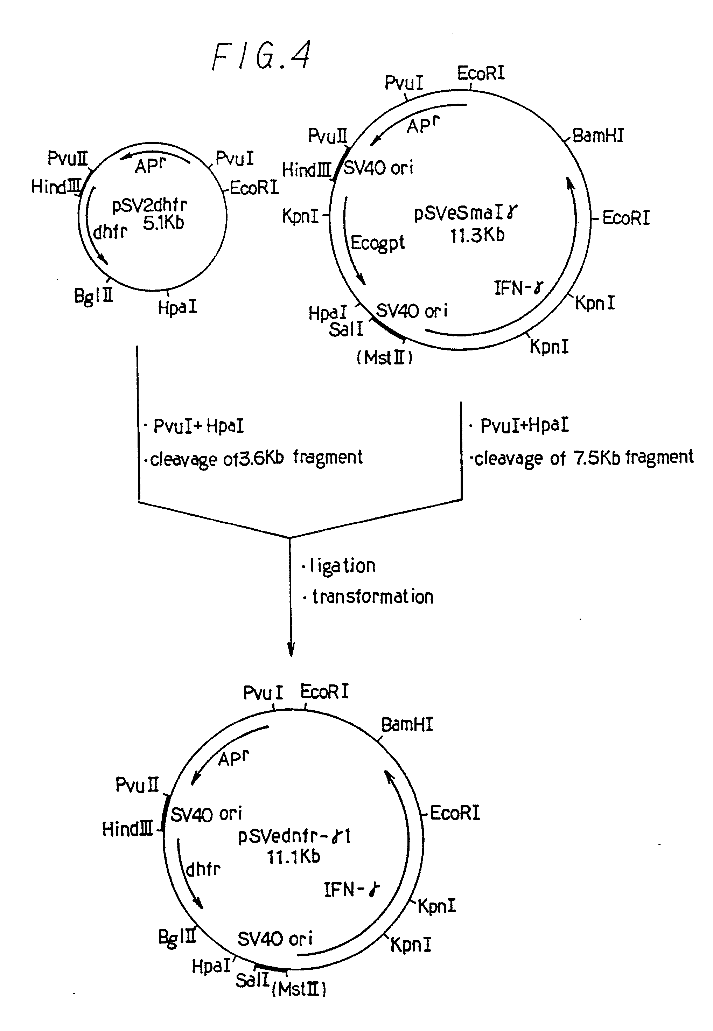

In the same manner as pSV2dhfr, 5 μg of pSVeSmalγ DNA was digested with Pvu I and Hpa I and the resultant was purified with agarose gel electrophoresis to give about 2.8 μg of about 7.5 Kb DNA fragment containing interferon-γ gene. Both DNA fragments were dissolved in 50 μl of a solution containing 20 mM Tris-HCl (pH 7.5), 6 mM MgCl$_2$, 5 mM dithiothreitol and 500 μM ATP and thereto 10 unit of T4 DNA ligase was added to conduct the linkage reaction at 4°C for 17 hours. By employing the obtained mixture of recombinant plasmid, E. coli HB101 was transformed in the usual way to give pSVedhfr-γ1 as shown in Fig. 4.

Example 5
[Preparation of CHO transformant with plasmid pSVedhfr-γ2]

According to the Calcium phosphate method [M.Wigler et al., Cell, 14, p 725 (1978)], transfection was conducted on CHO-KI (available from DAINIPPON PHARMACEUTICAL Co. Ltd., 2 × 10$^5$ cells) cultured on a dish (6 cm of diameter, Corning No. 25010) with 20 μg of pSVedhfr-γ2 DNA prepared in Example 3. After cultivation at 37°C for 48 hours, cultivation was further continued for 2 weeks on each selective medium of the transformant which is a nucleic acid defective α-MEM (made by GIBCO) containing 5 % dialyzed fetal bovine serum (made by GIBCO) together with ① 50 to 100 nM of MTX (selection condition, described as "MTX" in Table I), ② 78 μM of mycophenolic acid, 250 μg/ml of xanthine, 0.1 μg/ml of aminopterin, 5 μg/ml of thymidine and 25 μg/ml of adenine (selection condition, described as "MPA" in Tables I and 2), or ③ 78 μM of mycophenolic acid, 20 nM of MTX and 250 μg/ml of xanthine (selection condition, described as "MPA, MTX" in Tables I and 2), to form the colonies. Each colony was then transfered to 96-well multidish (Corning, No. 25860) and was grown confluently in the dish. After renewing the medium to nucleic acid defective α MEM containing 5 % dialyzed fetal bovine serum, cultivation was further continued at 37°C for 24 hours and the interferon-γ activity in the culture was measured.

Measurement of the antiviral activity of interferon-γ was assayed by inhibition of cytopathic effect of sindbis virus on human FL cell [C. Philips et al., Methods in Enzymology, 78, p 387 (1981)], employing international standard sample (Gg 23-901-530) as standard interferon-γ.

## Table 1

| Selective medium | Colony number/dish | Number of interferon-producing colony/20 colonies |
|---|---|---|
| MTX   50 nM | >1000 | 0 |
| MTX 100 nM | 10 | 0 |
| MPA   28 μM | 29 | 20 |
| MPA   78 μM, MTX 20 nM | 3 | 20 |

(Note) MTX: Methotrexate

MPA: Mycophenolic acid

## Table 2

| Interferon-producing cell line | Selective medium | Interferon unit/mℓ/day |
|---|---|---|
| γ2-P2 | MPA  78 μM | 1050 |
| γ2-P13 | MPA  78 μM | 525 |
| γ2-P14 | MPA  78 μM | 700 |
| γ2-PT5 | MPA  78 μM, MTX 20 nM | 700 |
| γ2-PT11 | MPA  78 μM, MTX 20 nM | 1400 |
| γ2-PT19 | MPA  78 μM, MTX 20 nM | 1050 |

The production of interferon-γ was examined on 20 colonies from each selective medium. As shown in Tables l and 2, interferon-producing cell line was not obtained by the selection with MTX alone, while the transformant which produced various amounts of interferon-γ was obtained by the selection with mycophenolic acid and the selection with mycophenolic acid and MTX.

Example 6
[Preparation of CHO transformant by co-transfection with plasmids pSVedhfr-γI and pSV2gpt]

pSVedhfr-γI prepared in Example 4 and pSV2gpt (ATCC 37l45) obtained from ATCC were mixed together in a molar ratio of 5 : I to make a total amount of 20 μg. By the same procedure as in Example 5, co-transfection was carried out and the transformant was selected with mycophenolic acid alone or with the selective medium containing both mycophenolic acid and MTX. The produced amount of interferon per 24 hours was measured on each transformant. The obtained results are shown in Table 3 for typical cell line.

## Table 3

| Interferon-producing cell line | Selective medium | Interferon unit/ml/day |
|---|---|---|
| γ1-P3 | MPA 78 μM | 525 |
| γ1-P9 | MPA 78 μM | 1050 |
| γ1-PT8 | MPA 78 μM, MTX 20 nM | 700 |
| γ1-PT14 | MPA 78 μM, MTX 20 nM | 1050 |
| γ1-PT19 | MPA 78 μM, MTX 20 nM | 1050 |

Example 7
[Gene amplification and increase in the interferon-γ production in CHO cells]

$10^3$ cells of each transformant obtained in Examples 5 and 6 were plated on a dish (diameter: l0 cm, Falcon, No. 3003) having l0 ml of nucleic acid defective α-MEM containing 50 or l00 nM of MTX and cultured at 37°C for 3 weeks with renewal of the medium every 3 or 4 days to form colonies. The obtained colonies were assayed for the interferon-γ activity as previously described. It was found that many transformants showed about l0 to 20 times higher activity than that of the unamplified transformant as shown in Table 4, which shows the gene amplification with MTX and the increase in the amount of the produced interferon-γ.

## Table 4

| Interferon-pro-ducing cell line | Culture condition | Amount of produced interferon (unit/ml/day) |
|---|---|---|
| γ2-P2-100 (29) | 100 nM MTX | 11200 |
| γ2-PT11-50 (6) | 50 nM MTX | 22400 |
| γ2-PT11-100 (16) | 100 nM MTX | 33600 |
| γ1-P9-100 (32) | 100 nM MTX | 22400 |
| γ1-PT19-50 (12) | 50 nM MTX | 11200 |
| γ1-PT19-100 (3) | 100 nM MTX | 33600 |

With respect to the comparison between two kinds of the selective condition of the transformant, i.e. the selection with mycophenolic acid alone and the selection with a combination of mycophenolic acid and MTX, it was shown that the latter condition produced more frequently the gene-amplified transformant and the existence of MTX in the selection of the transformant gave better results.

Copy number for interferon-γ gene was measured on these transformants. Measurement of the copy number for interferon-γ gene was conducted by DNA-DNA dot blot hybridization method [F. C. Kafatos et al., Nucleic Acids Res., 7, P l54l (l979)]. A chromosomal DNA was prepared as follows: i.e., the interferon-producing cell was cultured in l50 $cm^2$ flask (Corning, No. 25l20). After collecting the cells by the treatment with 0.02 % EDTA-0.25 % triptophan, they were washed twice with PBS solution [l0 mM $Na_2HPO_4$-$NaH_2PO_4$ (pH 7.2), l50 mM NaCl]. To the cells was added 20 ml of 0.5 M EDTA-0.5 % Sarcosyl® solution containing 2 mg of Proteinase K per $10^8$ cells and the mixture was heated at 50°C for 3 hours, followed by the treatment with phenol and the dialysis against TES solution [l0 mM Tris-HCl (pH 7.6), l mM EDTA, l0 mM NaCl]. After DNA was collected by the ethanol precipitation and dissolved in TES solution, RNA was decomposed with RNase A (l00 μg/ml). After the treatment with phenol, the dialysis and the ethanol precipitation, a sample of chromosomal DNA dissolved in water was prepared.

On the other hand, DNA of the interferon gene as a probe was prepared as follows: i.e., pSVeSmal-γ containing the interferon gene was digested in BamHI reaction solution by employing the restriction enzymes EcoRI and BamHI and the fragments were purified with agarose gel electrophoresis to give about 500 ng of EcoRI-BamHI fragment containing the 4th Exon. The obtained fragment was put in a nick translation kit (available from Amersham Co. Ltd.,) and thereto 5 mℓ of $[\alpha-^{32}P]$dCTP solution (800 Ci/mmoℓ, 10 mCi/mℓ). The mixure was reacted at 15°C for 3 hours to prepare probe DNA with $2.8 \times 10^7$ cpm.

One μg of the chromosomal DNA was spotted on nitrocellulose filter (Millipore Ltd.) and the filter was immersed successively into KOH solution for 5 minutes and then into IM Tris-HCℓ solution (pH 7.6) for 5 minutes each three times and was further immersed into $2 \times$ SSC solution (0.15 M NaCℓ, 0.015 M Na-citrate) for 1 minute. The filter was taken out and was subjected to baking procedure at 80°C for 1 hour. The filter having the denatured DNA was then put in a vinyl sack which contains 10 mℓ of a hybridization solution [2.5 mℓ of $20 \times$ SSPE (120 mM NaCℓ, 15 mM Na-citrate, 13 mM $KH_2PO_4$ , 1 mM EDTA), 1 mℓ of 50 × Denhardt solution (0.02 % BSA-fraction V, 0.02 % polyvinyl pyrrolidone, 0.02 % Ficoll), 0.1 mℓ of 10 % SDS and 6.4 mℓ of water] containing 2 mg of thermally denatured salmon DNA. After removing the air completely, the sack was sealed and the prehybridization was conducted at 65°C for 4 hours in advance. A mixture of probe DNA labeled with $^{32}$P-dCTP and 3 mℓ of a hybridization solution was put in another vinyl sack. The filter after prehybridization was immersed into the mixture to thoroughly soak the mixture into the filter. The air was completely removed and the vinyl sack was sealed to conduct the reaction at 65°C for a night. After completion of the reaction, the filter was taken out and was washed five times with each 500 mℓ of $2 \times$ SSC for 2 to 3 minutes. The filter was further washed three times with each 500 mℓ of $0.1 \times$ SSC at 65°C for 30 minutes. After removing water on the filter thoroughly with paper towel, the filter was exposed to X-ray on a X-ray film (RX-50 FUJI PHOTO FILM Co. Ltd.) for 4 days. Spot in the thus obtained negative film of one example of the amplified transformant showed that the interferon-γ gene was amplified not less than 20 times by the amlification procedure of DHFR cDNA with MTX treatment.

Example 8
[Transformation of MOPC with plasmid pSVedhfr-γ2 and increase in the produced amount of interferon-γ by the gene amplification procedure]

E. coli HB 101 transformed with pSVedhfr-γ2 was converted into the protoplast form according to Sandri-Goldin et al. [R. M. Sandri-Goldin et al., Mol. Cell, Biol., 1, P 743 (1981)]; i.e., to the cells proliferated to the early logarithmic phase in 25 mℓ of L medium containing 50 μg/mℓ of ampicillin, 125 μg/mℓ of chloramphenicol was added to amplify the plasmid. The cells were collected by centrifugation and was added with 1.25 mℓ of 20 % sucrose-0.05 M Tris-HCℓ (pH 8.0) and were cooled on ice. To the dispersion of the cells was added 0.25 mℓ of a solution of 0.25 M Tris-HCℓ (pH 8.0) in which 5 mg/mℓ of lysozyme was dissolved just before the addition. The mixture was allowed to stand for 5 minutes on ice. Thereto 0.5 mℓ of EDTA was further added and the mixture was allowed to stand for 5 minutes. After addition of 0.5 mℓ of 0.05 M Tris-HCℓ (pH 8.0), the cells were transfered into water bath at 37°C. After incubation for 10 minutes, the resultant was diluted into 10 mℓ of DME medium (Dulbecco's modified Eagle medium, made by Nissui Seiyaku) to prepare the cells in protoplast form. To 5 mℓ of the solution was added $6 \times 10^6$ cells of MOPC-31C (available from DAINIPPON PHARMACEUTICAL Co. Ltd.). After centrifugation at $500 \times g$ for 5 minutes, the resultant was dispersed into 2 mℓ of DME medium containing 50 % Polyethyleneglycol 4000 (made by Wako Pure Chemical Industries Ltd.). After centrifugation at $500 \times g$ for 3 minutes, 7 mℓ of DME medium was added, followed by the dispersion and centrifugation at $500 \times g$ for 5 minutes. The supernatant was removed and the cells were suspended in DME medium containing 10 % fetal bovine serum, which was transfered to 24-well multidish (Falcon, No. 3047). After cultivation for 48 hours, the same amount of DME medium containing 250 μg/mℓ of xanthine, 15 μg/mℓ of hypoxanthine, 25 μg/mℓ of mycophenolic acid, 40 nM of MTX and 10 % fetal bovine serum was added and the culture was continued for 2 weeks with renewal of the medium every 3 to 4 days to select transformant.

The transformant was cloned in a medium containing $10^4$ mouse peritoneal cells per 96-well multidish and $10^4$ cloned cells were dispersed in 10 mℓ of DME medium containing 100 or 200 nM of MTX and 10 % dialyzed fetal bovine serum to conduct cultivation on 24-well multidish for 3 weeks. After cloning the proliferating cells, the medium was renewed when the cell number reached $10^5$/mℓ and an amount of interferon-γ produced per day was measured. Many cloned cells with resistance to 100 nM MTX or 200 nM MTX produced more than 10 times larger amount of interferon. As the typical example, the amount of interferon produced by the transformant was 120 unit/mℓ/day while the transformant with the resistance to 100 nM MTX produced 2200 unit/mℓ/day and the transformant with the resistance to 200 nM MTX produced 3l50 unit/mℓ/day. From the above results, it was confirmed that the produced amount of interferon was increased also in MOPC-3IC by selecting the transformant with a combination of Ecogpt gene and mycophenolic acid and amplifying DHFR cDNA with MTX.

## Claims

1. A process for producing cells having a high productivity of a desired protein, which comprises:
   (1) transfecting a higher eucaryotic cell which contains a chromosomal DNA coding for dihydrofolate reductase sensitive to methotrexate and functions normally with a gene I coding for a desired protein, a gene II coding for dihydrofolate reductase derived from a wild type cell and a gene III coding for a protein necessary for the selection of a transformant, and
   (2) selecting the transformant in the presence of a growth inhibitor by using the properties of the protein encoded by said marker gene III and
   (3) culturing the transformant in the presence of a competitive inhibitor to the dihydrofolate reductase activity to amplify the gene I together with the amplification of the gene II.

2. The process of Claim 1, wherein the transfection is carried out by employing DNA in which the genes I, II and III are present on one and the same vector.

3. The process of Claim 1, wherein the transfection is carried out by employing DNA in which the genes I and II are present on one and the same vector and a vector DNA containing the gene III.

4. The process of Claim 1, 2 or 3, wherein the competitive inhibitor to the dihydrofolate reductase is methotrexate.

5. The process of Claim 1, 2, 3 or 4, wherein the gene III is the gene coding for guanine phosphoribosyl transferase, adenine phosphoribosyl transferase or thymidine kinase, or the gene coding for neomycin resistance.

6. The process of Claim 1, 2, 3, 4 or 5, wherein the gene II is cDNA coding for dihydrofolate reductase derived from mouse.

7. The process of Claim 1, 2, 3, 4, 5 or 6, wherein the transformant is selected in the presence of methotrexate.

8. The process of Claim 1, 2, 3, 4, 5, 6 or 7, wherein the higher eucaryotic cell which contains a chromosomal DNA coding for dihydrofolate reductase sensitive to methotrexate and functions normally, is CHO-KI or MOPC.

9. The process of Claim 1, 2, 3, 4, 5, 6, 7 or 8, wherein the gene I is the gene coding for human interferon-γ.

10. The process of Claim 5, wherein the gene III is the gene coding for guanine phosphoribosyl transferase derived from E. coli.

11. The process of Claim 2, wherein DNA in which the genes I, II and III are present on one and the same vector is pSVedhfr-γ2 (Fig. 3).

12. The process of Claim 3, wherein DNA in which the genes I and II are present on one and the same vector is pSVedhfr-γ1 (Fig.4), and the vector DNA containing the gene III is pSV2gpt (ATCC 37145)

13. A process for preparing a desired protein, which comprises:
    (1) transfecting a higher eucaryotic cell which contains a chromosomal DNA coding for dihydrofolate reductase sensitive to methotrexate and functions normally with a gene I coding for a desired protein, a gene II coding for dihydrofolate reductase derived from a wild type cell and a gene III coding for a protein necessary for the selection of a transformant,
    (2) selecting the transformant in the presence of a growth inhibitor by using the properties of the protein encoded by said marker gene III,

(3) culturing the transformant in the presence of a competitive inhibitor to the dihydrofolate reductase activity to amplify the gene I together with the amplification of the gene II, and
(4) culturing the thus obtained cell having a high productivity of the desired protein.

14. The process of Claim 13, wherein the transfection is carried out by employing DNA in which the genes I, II and III are present on one and the same vector.

15. The process of Claim 13, wherein the transfection is carried out by employing DNA in which the genes I and II are present on one and the same vector and a vector DNA containing the gene III.

16. The process of Claim 13, 14 or 15, wherein the competitive inhibitor to the dihydrofolate reductase is methotrexate.

17. The process of Claim 13, 14, 15 or 16, wherein the gene III is the gene coding for guanine phosphoribosyl transferase, adenine phosphoribosyl transferase or thymidine kinase, or the gene coding for neomycin resistance.

18. The process of Claim 13, 14, 15, 16 or 17, wherein the gene II is cDNA coding for dihydrofolate reductase derived from mouse.

19. The process of Claim 13, 14, 15, 16, 17 or 18, wherein the transformant is selected in the presence of methotrexate.

20. The process of Claim 13, 14, 15, 16, 17, 18 or 19, wherein the higher eucaryotic cell which contains a chromosomal DNA coding for dihydrofolate reductase sensitive to methotrexate and functions normally, is CHO-KI or MOPC.

21. The process of Claim 13, 14, 15, 16, 17, 18, 19 or 20, wherein the gene I is the gene coding for human interferon-$\gamma$.

22. The process of Claim 17, wherein the gene III is the gene coding for guanine phosphoribosyl transferase derived from E. coli.

23. The process of Claim 14, wherein DNA in which the genes I, II and III are present on one and the same vector is pSVedhfr-$\gamma$2 (Fig.3).

24. The process of Claim 15, wherein DNA in which the genes I and II are present on one and the same vector is pSVedhfr-$\gamma$1 (Fig.4), and the vector DNA containing the gene III is pSV2gpt (ATCC 37145)

**Revendications**

1. Procédé de production de cellules ayant une productivité élevée en une protéine désirée, dans lequel :
(1) on transfecte une cellule eucaryotique supérieure qui contient un ADN chromosomique codant pour la dihydrofolate réductase sensible au méthotréxate et qui fonctionne normalement avec un gène I codant pour une protéine désirée, un gène II codant pour la dihydrofolate réductase dérivée d'une cellule de type sauvage et un gène III codant pour une protéine nécessaire pour la sélection d'un transformant, et
(2) on sélectionne le transformant en présence d'un inhibiteur de croissance en utilisant les propriétés de la protéine encodée par ledit gène marqueur III, et
(3) on cultive le transformant en présence d'un inhibiteur compétitif de l'activité de la dihydrofolate réductase pour amplifier le gène I avec l'amplification du gène II.

2. Procédé de la revendication 1, dans lequel la transfection est réalisée en employant de l'ADN dans lequel les gènes I, II et III sont présents sur un seul et même vecteur.

3. Procédé de la revendication 1, dans lequel la transfection est réalisée en employant de l'ADN dans lequel les gènes I et II sont présents sur un seul et même vecteur et un ADN de vecteur contenant le gène III.

**4.** Procédé de la revendication 1, 2 ou 3, dans lequel l'inhibiteur compétitif vis-à-vis de la dihydrofolate réductase est le méthotrexate.

**5.** Procédé de la revendication 1, 2, 3, ou 4, où le gène III est le gène codant pour la guanine phosphoribosyl-transférase, l'adénine phosphoribosyl-transférase ou la thymidine-kinase ou le gène codant pour la résistance à la néomycine.

**6.** Procédé de la revendication 1, 2, 3, 4, ou 5, dans lequel le gène II est l'ADNc codant pour la dihydrofolate réductase dérivée de la souris.

**7.** Procédé de la revendication 1, 2, 3, 4, 5, ou 6 dans lequel le transformant est choisi en présence de méthotrexate.

**8.** Procédé de la revendication 1, 2, 3, 4, 5, 6, ou 7 dans lequel la cellule eucaryotique supérieure qui contient un ADN chromosomique codant pour la dihydrofolate réductase sensible au méthotrexat et qui fonctionne normalement est CHO-KI ou MOPC.

**9.** Procédé de la revendication 1, 2, 3, 4, 5, 6, 7, ou 8 dans lequel le gène I est le gène codant pour l'interféron γ humain.

**10.** Procédé de la revendication 5, dans lequel le gène III est le gène codant pour la guanine phosphoribosyl-transférase dérivée de E. coli.

**11.** Procédé de la revendication 2, dans lequel l'ADN dans lequel les gènes I, II et III sont présents sur le seul et même vecteur est pSVedhfr-γ2 (figure 3).

**12.** Procédé de la revendication 3, dans lequel l'ADN dans lequel les gènes I et II sont présents sur un seul et même vecteur est pSVedhfr-γ1 (figure 4) et l'ADN de vecteur contenant le gène III est pSV2gpt (ATCC 37145).

**13.** Procédé de préparation d'une protéine désirée, dans lequel :
(1) on transfecte une cellule eucaryotique supérieure qui contient un ADN chromosomique codant pour la dihydrofolate réductase sensible au méthotrexate et qui fonctionne normalement avec un gène I codant pour une protéine désirée, un gène II codant pour la dihydrofolate réductase dérivée d'une cellule de type sauvage et un gène III codant pour une protéine nécessaire pour la sélection d'un transformant, et
(2) on sélectionne le transformant en présence d'un inhibiteur de croissance en utilisant les propriétés de la protéine encodée par ledit gène marqueur III, et
(3) on cultive le transformant en présence d'un inhibiteur compétitif de l'activité de la dihydrofolate réductase pour amplifier le gène I avec l'amplification du gène II, et
(4) on cultive la cellule ainsi obtenue ayant une productivité élevée en protéine désirée.

**14.** Procédé de la revendication 13, dans lequel la transfection est réalisée en employant de l'ADN dans lequel les gènes I, II et III sont présents sur un seul et même vecteur.

**15.** Procédé de la revendication 13, dans lequel la transfection est réalisée en employant de l'ADN dans lequel les gènes I et II sont présents sur un seul et même vecteur et un ADN de vecteur contenant un gène III.

**16.** Procédé de la revendication 13, 14 ou 15, dans lequel l'inhibiteur compétitif vis-à-vis de la dihydrofolate réductase est le méthotrexate.

**17.** Procédé de la revendication 13, 14, 15 ou 16, dans lequel le gène III est le gène codant pour la guanine phosphoribosyl-transférase, l'adénine phosphoribosyl-transférase ou la thymidine-kinase ou le gène codant pour la résistance à la néomycine.

**18.** Procédé de la revendication 13, 14, 15, 16, ou 17, dans lequel le gène II est l'ADNc codant pour la dihydrofolate-réductase dérivée de la souris.

**19.** Procédé de la revendication 13, 14, 15, 16, 17 ou 18, dans lequel le transformant est sélectionné en présence de méthotrexate.

**20.** Procédé de la revendication 13, 14, 15, 16, 17, 18 ou 19, dans lequel la cellule eucaryotique supérieure qui contient un ADN chromosomique codant pour la dihydrofolate-réductase sensible au méthotrexate et fonctionne normalement est CHO-KI ou MOPC.

**21.** Procédé de la revendication 13, 14, 15, 16, 17, 18, 19 ou 20, dans lequel le gène I est le gène codant pour l'interféron γ humain.

**22.** Procédé de la revendication 17, dans lequel le gène III est le gène codant pour la guanine phosphoribosyl transférase dérivée d' E. coli.

**23.** Procédé de la revendication 14, dans lequel l'ADN dans lequel les gènes I, II et III sont présents sur un seul et même vecteur est pSVedhfr-γ2 (figure 3).

**24.** Procédé de la revendication 15, dans lequel l'ADN dans lequel les gènes I et II sont présents sur un seul et même vecteur est pSVedhfr-γ1 (figure 4) et l'ADN vecteur contenant le gène III est pSV2gpt (ATCC 37145).

**Patentansprüche**

**1.** Verfahren zur Herstellung von Zellen mit einer hohen Produktivität eines gewünschten Proteins, welches umfaßt:

(1) Transfection einer höheren eukaryotischen Zelle, welche enthält eine chromosomale DNA kodierend für Dihydrofolat-Reduktase empfindlich für Methotrexat und normal funktioniert mit einem Gen I kodierend für ein gewünschtes Protein, einem Gen II kodierend für die Dihydrofolat-Reduktase erhalten von einem Wildzellentyp und einem Gen III, kodierend für ein Protein notwendig für die Selektion eines Transformanden und
(2) Selektion des Transformanden in der Gegenwart eines Wachstumsinhibitors unter Verwendung der Eigenschaften des Proteins, kodiert durch genanntes Markergen III und
(3) Kultivieren des Transformanden in der Gegenwart eines konkurrierenden Inhibitors für die Dihydrofolat-Reduktase-Aktivität um das Gen I zu amplifizieren zusammen mit der Amplifikation des Gens II.

**2.** Verfahren nach Anspruch 1, worin die Transfection unter Verwendung von DNA in welcher die Gene I, II und III auf ein und demselben Vektor vorhanden sind, durchgeführt wird.

**3.** Verfahren nach Anspruch 1, worin die Transfection unter Verwendung von DNA, in welcher die Gene I und II auf ein und demselben Vektor vorhanden sind, und einer Vektor-DNA, die das Gen III enthält, durchgeführt wird.

**4.** Verfahren nach Anspruch 1, 2 oder 3, worin der konkurrierende Inhibitor zur Dihydrofolat-Reduktase Methotrexat ist.

**5.** Verfahren nach Anspruch 1, 2, 3 oder 4, worin das Gen III das Gen, kodierend für Guaninphosphoribosyl-Transferase, Adeninphosphoribosyl-Iransferase oder Thymidin-Kinase, oder das Gen kodierend für Neomycin-Resistenz ist.

**6.** Verfahren nach Anspruch 1, 2, 3, 4 oder 5, worin das Gen II cDNA kodierend für Dihydrofolat-Reduktase erhalten von Mäusen ist.

**7.** Verfahren von Anspruch 1, 2, 3, 4, 5 oder 6, worin der Transformand in der Gegenwart von Methotrexat ausgewählt ist.

**8.** Verfahren nach Anspruch 1, 2, 3, 4, 5, 6 oder 7, worin die höhere eukaryotische Zelle, welche enthält eine chromosomale DNA kodierend für Dihydrofolat-Reduktase empfindlich für Methotrexat und normal

funktioniert, CHO-KI oder MOPC ist.

9. Verfahren nach Anspruch 1, 2, 3, 4, 5, 6, 7 oder 8, worin das Gen I das Gen kodierend für menschliches Interferon-γ ist.

10. Verfahren nach Anspruch 5, worin das Gen III das Gen kodierend für Guaninphosphoribosyl-Transferase erhalten von Escherichia coli ist.

11. Verfahren nach Anspruch 2, worin die DNA, in welcher die Gene I, II und III auf ein und demselben Vektor vorhanden sind, pSVedhfr-γ2 (Fig. 3) ist.

12. Verfahren nach Anspruch 3, worin die DNA, in welcher die Gene I und II auf ein und demselben Vektor vorhanden sind, pSVedhfr-γ1 (Fig. 4) ist, und die Vektor-DNA, die das Gen III enthält, pSV2gpt (ATCC 37145) ist.

13. Verfahren zur Herstellung eines gewünschten Proteins, welches umfaßt:

(1) Transfection einer höheren eukaryotischen Zelle, welche enthält eine chromosomale DNA kodierend für Dihydrofolat-Reduktase empfindlich für Methotrexat und normal funktioniert mit einem Gen I, kodierend für ein gewünschtes Protein, einem Gen II kodierend für die Hydrofolat-Reduktase erhalten von einem Wildzellentyp und einem Gen III, kodierend für ein Protein notwendig für die Auswahl des Transformanden,
(2) Selektion des Transformanden in der Gegenwart eines Wachstumsinhibitors unter Verwendung der Eigenschaften des Proteins, kodiert durch genanntes Markergen III,
(3) Kultivieren des Transformanden in der Gegenwart eines konkurrierenden Inhibitors zur Dihydrofolat-Reduktase-Aktivität um das Gen I zu amplifizieren zusammen mit der Amplifikation des Gens II und
(4) Kultivieren der so erhaltenen Zelle mit einer hohen Produktivität des gewünschten Proteins.

14. Verfahren nach Anspruch 13, worin die Transfection unter Verwendung von DNA, in welcher die Gene I, II und III auf ein und demselben Vektor vorhanden sind, durchgeführt wird.

15. Verfahren nach Anspruch 13, worin die Transfection unter Verwendung von DNA ausgeführt wird, in welcher die Gene I und II auf ein und demselben Vektor vorhanden sind, und einer Vektor-DNA, die das Gen III enthält, durchgeführt wird.

16. Verfahren nach Anspruch 13, 14 oder 15, worin der konkurrierende Inhibitor zur Dihydrofolat-Reduktase Methotrexat ist.

17. Verfahren nach Anspruch 13, 14, 15 oder 16, worin das Gen III das Gen kodierend für Guaninphosphoribosyl-Transferase, Adeninphosphoribosyl-Transferaseoder Thymidin-Kinase oder das Gen kodierend für Neomycin-Resistenz ist.

18. Verfahren nach Anspruch 13, 14, 15, 16 oder 17, worin das Gen II cDNA kodierend für Dihydrofolat-Reduktase erhalten von Mäusen ist.

19. Verfahren nach Anspruch 13, 14, 15, 16, 17 oder 18, worin der Transformand in der Gegenwart von Methotrexat ausgewählt ist.

20. Verfahren nach Anspruch 13, 14, 15, 16, 17, 18 oder 19, worin die höhere eukaryotische Zelle, welche enthält eine chromosomale DNA kodierend für Dihydrofolat-Reduktase empfindlich für Methotrexat CHO-KI oder MOPC ist.

21. Verfahren nach Anspruch 13, 14, 15, 16, 17, 18, 19 oder 20, worin das Gen I das Gen kodierend für menschliches Interferon-γ ist.

22. Verfahren nach Anspruch 17, worin das Gen III das Gen kodierend für Guaninphosphoribosyl-Transferase erhalten von Escherichia coli ist.

**23.** Verfahren nach Anspruch 14, worin die DNA, in welcher die Gene I, II und III auf ein und demselben Vektor vorhanden sind, pSVedhfr-γ2 (Fig. 3) ist.

**24.** Verfahren nach Anspruch 15, worin die DNA, in welcher die Gene I und II auf ein und demselben Vektor vorhanden sind, pSVedhfr-γ1 (Fig. 4) ist, und die Vektor DNA, die das Gen III enthält, pSV2gpt (ATCC 37145) ist.

# FIG. 1

FIG.2a

EP 0 223 230 B1

17

# F I G. 2b

# FIG.3

# FIG.4